# EUROPEAN PATENT SPECIFICATION

(11) **EP 2 073 759 B1**
(45) Date of publication and mention of the grant of the patent: **20.03.2013**
(21) Application number: 07844023.7
(22) Date of filing: 09.10.2007
(51) Int. Cl.: A61F 2/38

(54) **Knee prosthesis kit**
Knieprothesensatz
Kit de prothèse du genou

(30) Priority: 13.10.2006 US 829432 P; 13.10.2006 US 829430 P; 21.09.2007 US 859425
(43) Date of publication of application: 01.07.2009
(73) Proprietor: DePuy Products, Inc., Warsaw, IN 46581 (US)
(72) Inventor: BONITATI, John A., Warsaw IN 46580 (US); JACOBS, Andrew, Fort Wayne IN 46804 (US); THOMAS, Kyle, Fort Wayne IN 46814 (US); BERTAZZONI, Mario, Vigevano 27029 (IT); KULKARNI, Nikhil, St. Paul, MN 55129 (US)
(74) Representative: Belcher, Simon James
(86) International application number: PCT/US2007/080797
(87) International publication number: WO 2008/048822

(56) References cited:
- EP-A- 0 956 836
- EP-A- 1 702 590
- US-A- 5 879 391
- US-A1- 2002 120 341

## Description

The present invention relates generally to a knee prosthesis kit.

Movement (e.g., flexion and extension) of the natural human knee involves movements of the femur and the tibia. Specifically, during flexion and extension, the distal end of the femur and the proximal end of the tibia articulate relative to one another through a series of complex movements. Damage (e.g., trauma) or disease can deteriorate the bones, articular cartilage, and ligaments of the knee, which can ultimately affect the ability of the natural knee to function in such a manner. As a result, knee prostheses have been developed and implanted into surgically prepared ends of the femur and tibia.

A typical knee prosthesis for a total knee replacement, for example, includes a tibial component or tibial tray coupled to the patient's tibia, a femoral component coupled to the patient's femur, and a bearing component (or tibial insert) positioned between the tibial tray and the femoral component and including a bearing surface to accommodate the condyles of the femoral component. In some situations, it may be desirable that the tibial insert rotate relative to the tibial tray. Such rotation more closely replicates the motion of the patient's natural anatomy. In other cases, however, it may be desirable to prevent the tibial insert from rotating relative to the tibial tray. For example, various ligaments which support the knee may be compromised or damaged. In such a case, rotation of the tibial insert relative to the tibial tray may create an unstable knee. As such, a surgeon will decide on a case-by- case basis whether to use a rotating or non-rotating tibial assembly. This decision may be made pre-operatively or intra-operatively, for example. Additionally, it may be desirable to change a rotating tibial insert to a non-rotating tibial insert during a revision type surgery, for example.

US-5879391 discloses a knee prosthesis which includes femoral and tibial components. Each of the femoral and tibial components includes a stem. Each of the stems engages the main body portion of its respective component in such a way that the stem can be fitted at a number of different spaced apart positions along a slot-like aperture in the component.

The present invention provides a knee prosthesis kit as defined in claim 1.

Optional features of the invention are specified in the dependent claims.

The detailed description particularly refers to the following figures, in which:
FIG. 1 is a bottom perspective view of an orthopaedic prosthesis assembly including a tibial tray and an adjustable stem;
FIG. 2 is a top perspective view of the orthopaedic prosthesis assembly of FIG. 1;
FIG. 3 is a bottom plan view of another embodiment of the orthopaedic prosthesis assembly of FIG. 1;
FIG. 4 is a bottom perspective view of a femoral component of an orthopaedic prosthesis assembly; and
FIG. 5 is front elevation view of the femoral component of FIG. 4.

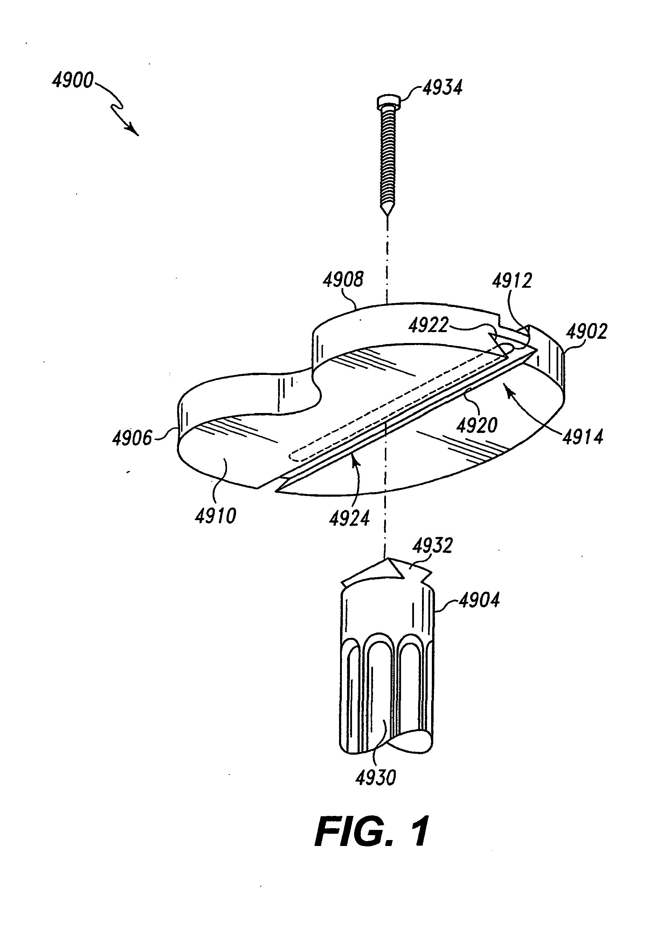

Referring to the drawings, FIGS. 1 to 3 show a prosthetic knee system 4900 which includes a tibial tray 4902 and an adjustable stem 4904. The tibial tray 4902 and adjustable stem 4904 are illustratively formed from an implantable metallic material, but may be formed from other materials, such as a ceramic material, a polymer material, a bio-engineered material, or the like, in other embodiments.

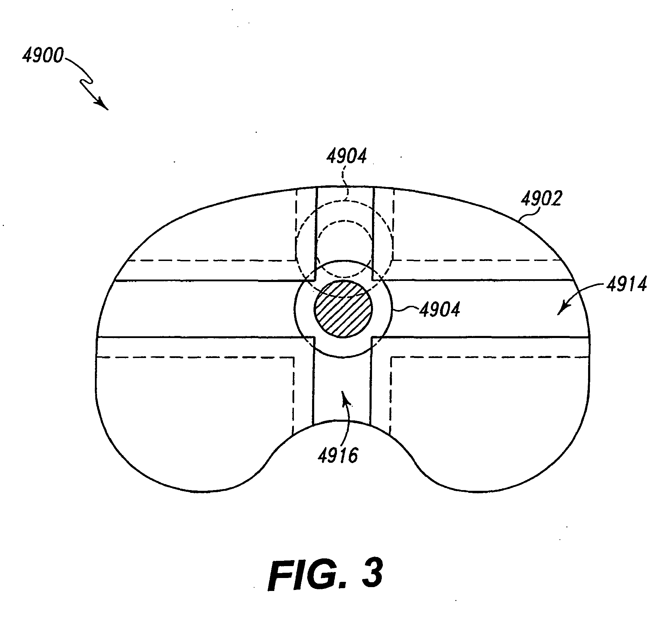

The tibial tray 4902 includes a platform 4906 having an upper surface 4908 and a bottom surface 4910. The tibial tray 4902 also includes a recessed elongated opening 4912 in the upper surface 4908 and a recessed guide track 4914 in the bottom surface 4910. The guide track 4914 is defined in the bottom surface 4910 in a medial/lateral direction, but may be defined in other directions in other embodiments. Additionally, although the illustrative tibial tray 4902 includes a single guide track 4914, the tibial tray 4902 may include additional guide tracks in other embodiments. For example, as illustrated in FIG. 3, the tibial tray 4902 may include a guide track 4916 that is defined in the bottom surface 4910 in an anterior/posterior direction such that the position of the stem 4904 relative to the tibial tray 4902 may be configured in either a medial/lateral direction or an anterior/posterior direction

The illustrative guide track 4914 is substantially dovetailed and is configured to receive a portion of the stem 4902. The guide track 4914 includes an anterior sidewall 4920 and a posterior sidewall 4922. The sidewalls 4920, 4922 are inwardly sloped to define an opening 4924 in the bottom surface 4910 therebetween. However, in other embodiments, the guide track 4914 may have other shapes such as a substantially rectangular shape. The illustrative guide track 4914 is an open track having open ends. However, in other embodiments, the guide track 4914 may be a closed track having one or both ends closed.

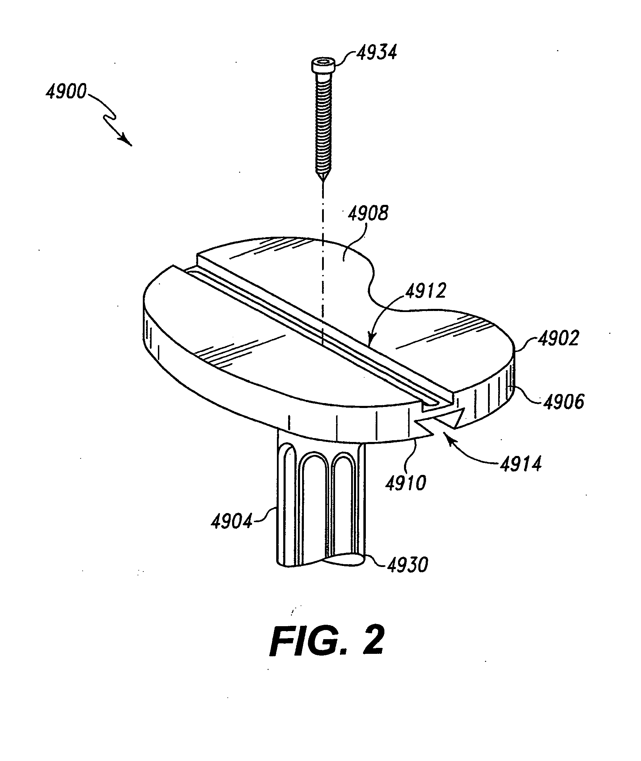

The stem 4904 includes an elongated shaft 4930 and a mounting end 4932 defined on a proximal end of the elongated shaft 4930. The mounting end 4932 has a shape corresponding to the shape of the guide track 4914 such that the mounting end 4932 may be received therein. In the illustrative embodiments of FIGS. 1 and 2, the mounting end 4932 has a substantially dovetail shape, but may have other shapes corresponding to the shape of the guide track 4914 in other embodiments. The mounting end 4932 is sized to be received in the guide track 4914. Once so received, the stem 4904 may then be slid or otherwise positioned to the desired location along the guide track 4914. Once positioned in the desired location, the stem 4904 may be secured to the tibial tray 4902 via use of a fastener 4934, which may be inserted through the elongated opening 4912 defined in the upper surface 4908 of the tibial tray 4902. Because the elongated opening 4912 is recessed in the upper surface 4908, the head of the fastener 4934 is positioned at or below the upper surface 4908.

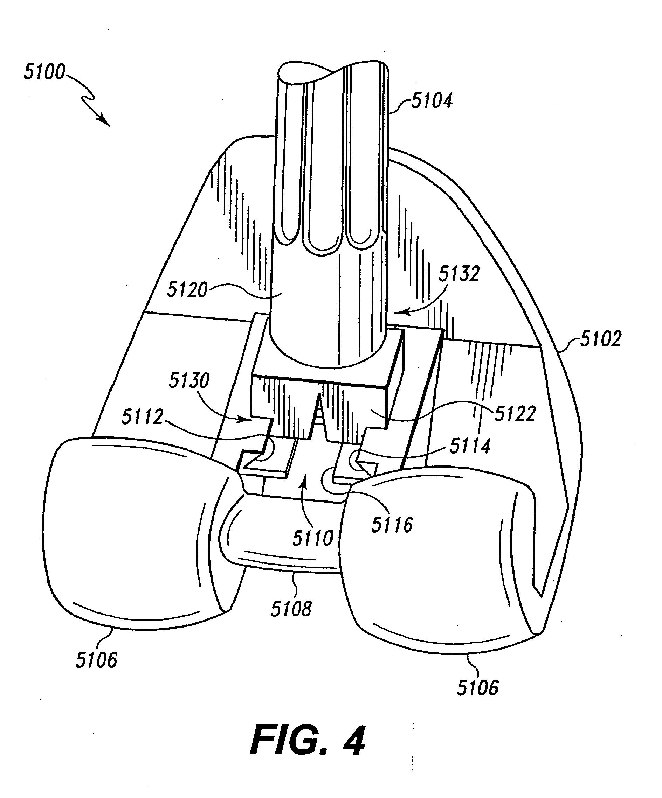

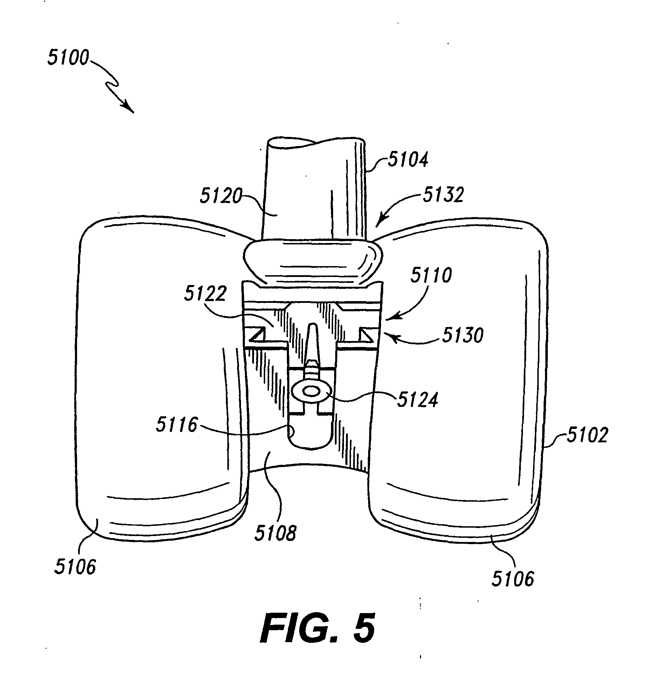

FIGS. 4 and 5 show a prosthetic knee system 5100 which includes a femoral component 5102, an stem 5104, and an adaptor 5122 coupled to the femoral component 5102. The femoral component 5102, the stem 5104, and the adaptor 5122 are illustratively formed from an implantable metallic material, but may be formed from other materials, such as a ceramic material, a polymer material, a bio-engineered material, or the like, in other embodiments.

The femoral component 5102 is configured to be coupled to a surgically-prepared surface of the distal end of a patient's femur (not shown). When the femoral component 5102 is coupled to the patient's femur and the stem 5104 and the adaptor 5122 are coupled to the femoral component 5102 as discussed below, the stem 5104 is embedded in the patient's bone. The femoral component 5102 may be secured to the patient's femur via use of bone adhesive or other attachment means. The femoral component 5102 includes a pair of condyles 5106. In use, the condyles 5106 replace the natural condyles of the patient's femur and are configured to articulate on the proximal end of the patient's natural or surgically-prepared tibia.

The femoral component 5102 includes a platform 5108 defined between the condyles 5106. The platform 5108 includes a guide track 5110 defined therein. The illustrative guide track 5110 is substantially dovetailed and is configured to receive a portion of the adaptor 5122. The guide track 5110 includes an anterior sidewall 5112 and a posterior sidewall 5114. The sidewalls 5112, 5114 are inwardly sloped to define an opening 5116 in the platform 5108 therebetween. However, in other embodiments, the guide track 5110 may have other shapes such as a substantially rectangular shape. The illustrative guide track 5110 is an open track having open ends. However, in other embodiments, the guide track 5110 may be a closed track having one or both ends closed.

The adaptor 5122 includes a mounting end 5130 configured to be received in the guide track 5110. That is, the mounting end 5130 has a shape corresponding to the shape of the guide track 5110 such that the mounting end 5130 may be received therein. In the illustrative embodiments of FIGS. 4 and 5, the mounting end 5130 has a substantially dovetail shape, but may have other shapes corresponding to the shape of the guide track 5110 in other embodiments. The mounting end 5130 of the adaptor 5122 is sized to be received in the guide track 5110. Once so received, the adaptor 5122 (and the stem 5104) may be slid or otherwise positioned to the desired location along the guide track 5110. Once positioned in the desired location, the adaptor 5122 may be secured to the femoral component 5102 via use of a fastener 5124, which may be inserted through the opening 5116 defined in the platform 5108 of the femoral component 5102.

According to the invention, the adaptor 5122 is integral with the stem 5104 and may be used with the tibial tray 4902 illustrated in and described above with reference to FIG. 1.

Prosthetic knee systems and assemblies may include a single tibial tray, non-rotating or fixed tibial insert, and rotating tibial insert. The invention provides prosthetic knee systems having one or more tibial trays, one or more tibial inserts, and/or one or more locking mechanisms or other components associated with the aforementioned tray(s) and insert(s). A first combination of the components of such a prosthetic knee system provides a rotating tibial assembly whereby the tibial insert is able to rotate about an axis relative to the tibial tray. A second combination of the components such a prosthetic knee system provides a non-rotating or fixed knee assembly whereby the tibial insert is fixed relative to the tibial tray and is not able to rotate about the axis. As such, the invention includes prosthetic knee systems including components which may be arranged to provide for both a rotating knee assembly and a non-rotating knee assembly.

Features for coupling tibial trays and tibial inserts together in order to prevent rotation of the tibial insert relative to the tibial tray, to reduce or minimize micromotion between the tibial insert and the tibial tray, and/or to prevent lift-off of the tibial insert relative to the tibial tray, for example, may be located on or within each tibial insert and/or tibial tray. Alternatively, these features may be embodied by components separate from the tibial insert and tibial trays.

## Claims

1. A knee prosthesis kit comprising:
a femoral component (5102) configured to be coupled to a surgically-prepared surface of the distal end of a femur, the femoral component having a pair of spaced apart condyles (5106) and a platform (5108) defined between them, the platform including a femoral component guide track (5110) defined therein for receiving the mounting end of a stem, and an elongated opening (5116) for accessing a threaded aperture in the stem to secure the stem to the femoral component in any one of a number of selectable positions along the femoral component guide track, and
a tibial tray (4902) configured to be coupled to a surgically-prepared surface of the proximal end of a tibia, the tibial tray including a platform (4906) having an upper surface (4908) and a bottom surface (4910) and a tibial tray guide track (4914) extending downwardly from the bottom surface for receiving the mounting end of a stem which can be secured to the tibial tray in any one of a number of selectable positions along the tibial tray guide track,
wherein the kit includes a stem (4902, 5104) which can be coupled separately to each of the femoral component and the tibial tray, the stem having a mounting end (4932, 5130) and a threaded aperture defined in a distal end of the mounting end, **characterised in that**
(a) the mounting end (4932, 5130) of the stem can be received in the femoral component guide track (5110) on the femoral component platform (5108),
(b) the mounting end (4932, 5130) of the stem can be received in the tibial tray guide track (4914) in the tibial tray (4902).

2. The orthopaedic prosthesis of claim 1, in which the guide track (5110) of the femoral component (5102) and the mounting end (5130) of the stem (5104) have corresponding dovetail shapes.

3. The orthopaedic prosthesis of claim 1, further comprising a fastener (5124) which can be received in the elongated opening (5116) and the threaded aperture to secure the stem (5104) to the femoral component (5102).

## Patentansprüche

1. Knieprothesensatz, umfassend:
eine Femurkomponente (5102), die zur Kopplung mit einer chirurgisch vorbereiteten Fläche des distalen Endes eines Femurs konfiguriert ist, wobei die Femurkomponente ein Paar von einander beabstandete Kondylen (5106) und eine dazwischen definierte Plattform (5108) aufweist, wobei die Plattform eine Femurkomponentenführungsschiene (5110), die darin zur Aufnahme des Befestigungsendes eines Schaftes definiert ist, und eine längliche Öffnung (5116) für einen Zugang zu einer Gewindeöffnung in dem Schaft zum Sichern des Schaftes an der Femurkomponente in irgendeiner einer Anzahl von wählbaren Positionen entlang der Femurkomponentenführungsschiene enthält, und
ein Tibiaplateau (4902), das zur Kopplung mit einer chirurgisch vorbereiteten Fläche des proximalen Endes einer Tibia konfiguriert ist, wobei das Tibiaplateau eine Plattform (4906) mit einer oberen Fläche (4908) und einer unteren Fläche (4910) und eine Tibiaplateauführungsschiene (4914) enthält, die sich von der unteren Fläche nach unten zur Aufnahme des Befestigungsendes eines Schaftes erstreckt, der an dem Tibiaplateau in irgendeiner einer Anzahl von wählbaren Positionen entlang der Tibiaplateauführungsschiene gesichert werden kann,
wobei der Satz einen Schaft (4902, 5104) enthält, der separat mit jedem von der Femurkomponente und dem Tibiaplateau gekoppelt werden kann, wobei der Schaft ein Befestigungsende (4932, 5130) und eine Gewindeöffnung aufweist, die in einem distalen Ende des Befestigungsendes definiert ist, **dadurch gekennzeichnet, dass**
(a) das Befestigungsende (4932, 5130) des Schafts in der Femurkomponentenführungsschiene (5110) an der Femurkomponentenplattform (5108) aufgenommen werden kann und
(b) das Befestigungsende (4932, 5130) des Schafts in der Tibiaplateauführungsschiene (4914) in dem Tibiaplateau (4902) aufgenommen werden kann.

2. Orthopädische Prothese nach Anspruch 1, wobei die Führungsschiene (5110) der Femurkomponente (5102) und das Befestigungsende (5130) des Schafts (5104) korrespondierende Schwalbenschwanz-Formen aufweisen.

3. Orthopädische Prothese nach Anspruch 1, ferner umfassend ein Befestigungselement (5124), das in der länglichen Öffnung (5116) und der Gewindeöffnung zur Sicherung des Schafts (5104) an der Femurkomponente (5102) aufgenommen werden kann.

## Revendications

1. Kit de prothèse de genou comprenant :
un composant fémoral (5102) configuré de façon à être couplé à une surface préparée de manière chirurgicale de l'extrémité distale d'un fémur, le composant fémoral présentant une paire de condyles espacés (5106) et une plate-forme (5108) définie entre eux, la plate-forme comprenant une voie de guidage de composant fémoral (5110) définie à l'intérieur et destinée à recevoir l'extrémité de montage d'une tige, et une ouverture allongée (5116) qui permet d'accéder à une ouverture filetée dans la tige de façon à fixer la tige sur le composant fémoral selon n'importe laquelle d'un certain nombre de positions qui peuvent être sélectionnées le long de la voie de guidage de composant fémoral ; et
un plateau tibial (4902) configuré de façon à être couplé à une surface préparée de manière chirurgicale de l'extrémité proximale d'un tibia, le plateau tibial comprenant une plate-forme (4906) qui présente une surface supérieure (4908) et une surface inférieure (4910) et une voie de piste de plateau tibial (4914) qui s'étend vers le bas à partir de la surface inférieure et destinée à recevoir l'extrémité de montage d'une tige qui peut être fixée sur le plateau tibial selon n'importe laquelle d'un certain nombre de positions qui peuvent être sélectionnées le long de la voie de piste de plateau tibial ;
dans lequel le kit comprend une tige (4902, 5104) qui peut être couplée de manière séparée à chacun du composant fémoral et du plateau tibial, la tige présentant une extrémité de montage (4932, 5130) et une ouverture filetée définie dans une extrémité distale de l'extrémité de montage, **caractérisé en ce que** :
(a) l'extrémité de montage (4932, 5130) de la tige peut être reçue dans la voie de piste de composant fémoral (5110) sur la plate-forme de composant fémoral (5108) ; et
(b) l'extrémité de montage (4932, 5130) de la tige peut être reçue dans la voie de piste de plateau tibial (4914) dans le plateau tibial (4902).

2. Prothèse orthopédique selon la revendication 1, dans laquelle la voie de piste (5110) du composant fémoral (5102) et l'extrémité de montage (5130) de la tige (5104) présentent des formes en queue d'aronde correspondantes.

3. Prothèse orthopédique selon la revendication 1, comprenant en outre un dispositif de fixation (5124) qui peut être reçu dans l'ouverture allongée (5116) et dans l'ouverture filetée de façon à fixer la tige (5104) sur le composant fémoral (5102).
